# EUROPEAN PATENT SPECIFICATION

(11) **EP 2 022 520 B1**
(45) Date of publication and mention of the grant of the patent: **07.03.2018**
(21) Application number: 08160785.5
(22) Date of filing: 21.07.2008
(51) Int. Cl.: A61M 5/145

(54) **Drug infusion device equipped with a syringe having a pair of tabs**
Injektionsvorrichtung mit einer Spritze, die Befestigungslaschen enthält
Disposition d'infusion comprenant une seringue ayant des languettes de connection

(30) Priority: 23.07.2007 IT TO20070540
(43) Date of publication of application: 11.02.2009
(73) Proprietor: CANE' S.P.A., 10098 Rivoli (TO) (IT)
(72) Inventor: Cane', Mario, 10095 Collegno (Torino) (IT)
(74) Representative: Robba, Pierpaolo

(56) References cited:
- EP-A- 1 776 973
- US-A- 6 080 136
- US-A1- 2003 060 754
- US-A1- 2006 100 581

## Description

The present invention concerns a, syringe for drug infusion devices and a drug infusion device equipped therewith.

In the field of electro-medical apparatuses, electronic devices are known for prolonged drug infusion through a disposable syringe, generally made of moulded plastics.

More particularly, compact and portable drug infusion devices are known, for use by patients that can walk and perform a substantially normal physical activity.

An example of compact and portable infusion device is disclosed in EP 1078643 in the name of the same Applicant.

Certain known portable infusion devices, such as the one described in the patent application mentioned above, comprise a connecting member on which a syringe filled with the liquid drug can be secured, the drug progressively coming out from the syringe under the push of a slide or pusher operated by the motor of the infusion device.

In the known devices, coupling of the syringe with the pump generally takes place by means of a ring nut which is arranged on the base of the infusion device and to which the syringe may be coupled through a rotary movement.

To this end, in the known devices the syringes are equipped at their base with a pair of tabs that are inserted into the ring nut of the infusion device. Moreover, each tab preferably has a notch that can accommodate a corresponding pin provided inside the slot defined by the ring nut in order to receive the tabs, so that the faint click produced by the pin when entering the notch indicates to the user that the syringe is correctly positioned.

US 6 080 136 discloses an angiographic syringe having at its proximal end on the exterior surface thereof two diametrally opposed flange or lug members, for engaging and locking through rotation in either clockwise or counterclockwise direction the syringe to an adapter for interconnecting the syringe with an injector.

EP 1 776 973 discloses a system for coupling a syringe with a power injector wherein the syringe is provided with a radially outwardly extending flange having a polygonal outer edge of octagonal shape; a locking mechanism is provided on the injector which can be operated to lock the syringe once the flange has been inserted through an axial movement in a complementary seat within the injector body.

US 2006/100581 discloses a reservoir comprising connection means for securing the reservoir in a chamber of an infusion device; the connection means are located at the closed end of the reservoir and comprise elevations disposed on the side surface of the body portion of the reservoir corresponding with a threading disposed in the chamber and allow the reservoir to be screwed into place in the chamber of the infusion device.

US 2003/060754 discloses a syringe rotatably mountable on a front wall of an injector housing by a first-quick release mechanism; the front wall of the injector comprises an opening provided with a pair of upper and lower slots through which respective upper and lower retaining flanges of the syringe may pass as the rearward end of the syringe is inserted in the opening; projecting stops are provided in the opening of the injector housing to limit the rotation of the syringe during mounting thereof.

Yet, a drawback of the known devices is that, even though on the one hand the interference between the pin of the ring nut and the notch in the syringe tab allows indicating the attainment of the proper position and avoiding fortuitous movements of the syringe, on the other hand such interference is not sufficient to prevent a further rotation of the syringe itself by the user, so that the risk exists of disconnecting again said syringe from the ring nut of the infusion device.

The gravity of such a drawback is immediately apparent when one considers that the users of such infusion devices are ill and often aged people, who in some cases have difficulty in coordinating movements.

Thus, it is an object of the present invention to overcome the drawbacks of the known devices, by providing a solution to the problem of how to prevent the device from being used with a syringe that is not properly connected.

The above and other objects are achieved by means of the syringe and the drug infusion device according to the invention, as claimed in the appended claims.

Thanks to the provision in the syringe according to the invention of at least one locator abutting against the ring nut of the infusion device, the risks associated with an excessive syringe rotation relative to said ring nut can be eliminated.

Advantageously, no modification of the ring nut of the infusion device is required, so that the syringe according to the invention can be used with already existing infusion devices, without requiring any modification thereof.

It is further clear that the solution offered by the present invention to the above drawbacks is extremely simple and cheap.

A preferred embodiment of the invention, given by way of non-limiting example, will be described in detail hereinafter, with reference to the accompanying drawings, in which:
- Fig. 1 is a perspective view of the syringe according to the invention;
- Fig. 2 is a top view of the syringe depicted in Fig. 1;
- Fig. 3 is a perspective view of an infusion device incorporating the syringe depicted in Fig. 1;
- Fig. 4 is a top view of the infusion device depicted in Fig. 3;
- Fig. 5 is a top view of the syringe of the invention, according to a variant embodiment.

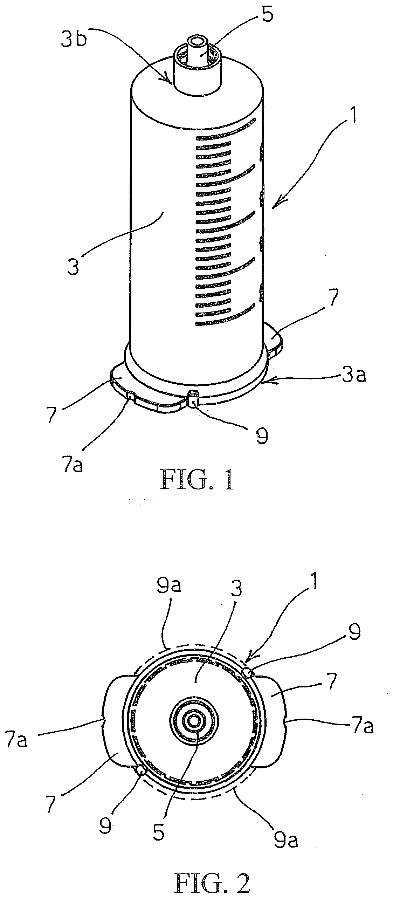

Referring to Figures 1 and 2, there is shown a syringe 1 according to the invention, comprising a substantially cylindrical body 3, preferably graduated, which may be filled with a liquid drug. Said body 3 has, at a first end or base 3a, a pair of tabs 7 for coupling the syringe to an infusion device and, at the opposite end 3b, a terminal 5 for mounting a needle or a cannula for administering said liquid drug.

As described above, in conventional manner, each tab 7 has a notch 7a arranged to cooperate with a corresponding pin provided in the ring nut of the known infusion devices.

According to the invention, syringe 1 further has, in correspondence of base 3a, at least one locator 9 arranged to abut against the side wall of said ring nut.

Advantageously, for economy reasons, said at least one locator 9 is defined by a corresponding stop projection or tooth, e.g. cylindrical, radially defined with respect to the base of syringe 1.

However, said locator could be defined by a corresponding projecting edge circumferentially extending between tabs 7, as shown by dashed line 9a in Fig. 2.

Preferably, in the described embodiment, syringe 1 has a pair of stop teeth 9 located at diametrically opposite positions along the periphery of base 3a of body 3.

Preferably said stop teeth 9 are integrally formed with body 3 of syringe 1, directly during the syringe moulding. However, they could even be subsequently applied, for instance glued or soldered, to conventional syringes.

Preferably, tabs 7 of syringe 1 are sized such that each stop tooth 9 is adjacent to the corresponding tab 7.

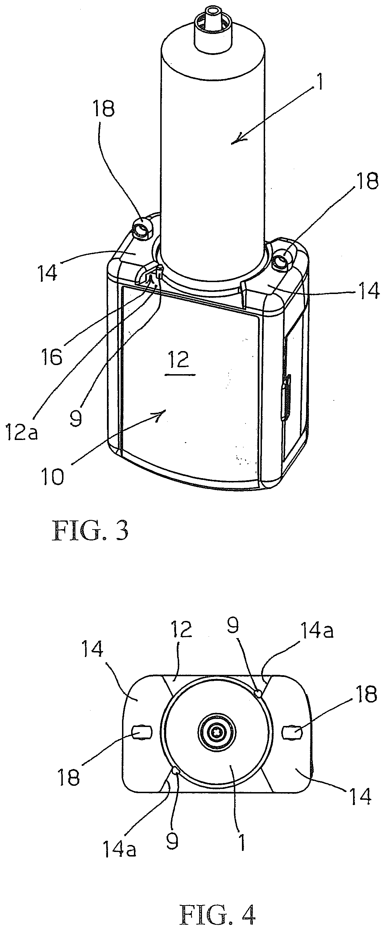

The function of stop teeth 9 becomes even more apparent from Figs. 3 and 4, which show syringe 1 mounted on an infusion device 10.

Said infusion device 10 includes a housing 12 which accommodates an electric motor and corresponding mechanical means for axially displacing a pusher that pushes the plunger of syringe 1 thereby gradually delivering the drug contained in the syringe, as well as an electronic control device arranged to adjust the rate and the duration of drug delivery and other parameters of the infusion device.

A ring nut 14 is provided in correspondence with one base 12a of housing 12 for coupling syringe 1 with infusion device 10. To this aim, said ring nut 14 is interrupted to allow inserting syringe 1 on base 12a of housing 12, and it defines with said base 12a a pair of diametrically opposite slots 16 where tabs 7 of syringe 1 can be inserted by rotating body 3 of syringe 1 about its axis.

Ring nut 14 further has a pair of eyes 18, which are supported by corresponding fastening pins passing through slots 16 and through which a strap can pass to make the infusion device transportation easier by allowing the device to be hung for instance on the patient's neck.

Syringe 1, once it has been inserted on base 12a of housing 12, is rotated clockwise as long as the pins supporting eyes 18 engage notches 7a in tabs 7.

Figs. 3 and 4 make it apparent that, thanks to the features of the present invention, when syringe 1 is properly positioned, with tabs 7 properly located in slots 16, stop teeth 9 abut against side walls 14a of ring nut 14, thereby preventing any further clockwise rotation of body 3 of syringe 1.

In this way, the invention attains the desired aims, since it provides a syringe equipped with stopping means for preventing a further rotation of the syringe itself beyond the correct position, whereby the risk of disconnecting the syringe from the ring nut of the infusion device is avoided.

It is to be appreciated that, whilst according to the prior art the syringe could be indifferently rotated in either direction for being coupled with the ring nut of the infusion device, according to the invention the rotation direction is univocally determined by the position of stop teeth 9, which must abut against walls 14a of ring nut 14 when tabs 7 of the syringe are properly located below said ring nut. More particularly, in the embodiment shown, syringe 1 is to be rotated in clockwise direction. However, teeth 9 could be located on the other side of the respective tab 7, so that the syringe is to be inserted into the ring nut through a counterclockwise rotation.

Consequently, whilst according to the prior art the tabs were to have a symmetrical profile relative to a plane passing through notch 7a, such a constraint is superfluous in the syringe according to the invention.

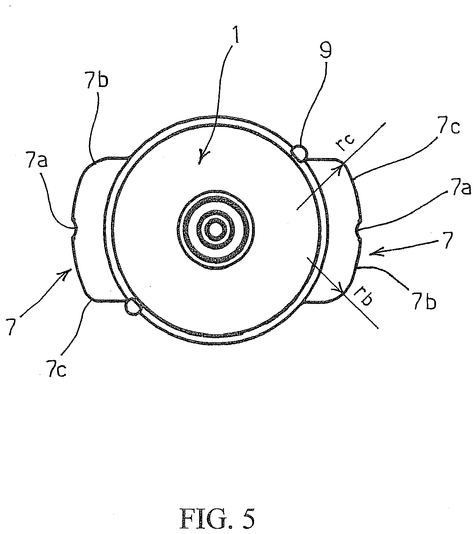

In this respect, Fig. 5 shows a variant embodiment of the syringe according to the invention having asymmetrical tabs 7.

According to such a variant embodiment, each tab 7 has a draft portion 7b, distal from stop tooth 9, with a greater radius of curvature r_{b} and therefore with a more smoothed and rounded profile, and a stopping portion 7c, proximal to stop tooth 9, with a smaller radius of curvature r_{c} and therefore with a sharper profile.

It is clear that the skilled in the art can envisage several variants of the stopping means, without departing from the principle of the invention.

## Claims

1. A combination of a drug infusion device (10) and a syringe (1),
- wherein the syringe (1) comprises:
- a substantially cylindrical body (3) which may be filled with a liquid drug, said body (3) having at a first end or base (3a), a pair of tabs (7) for coupling the syringe to infusion device (1) through rotation and, at the opposite end (3b), a terminal (5) for mounting a needle or a cannula for administering said liquid drug;
- wherein the infusion device (10) comprises:
- a housing (12) which accommodates an electric motor and corresponding mechanical means for axially displacing a pusher that pushes the plunger of syringe (1) mounted in said infusion device (10) and containing a drug so as to gradually deliver said drug contained in the syringe, as well as an electronic control device arranged to adjust the rate and the duration of drug delivery and other parameters of the infusion device;
- a ring nut (14) provided in correspondence with one base (12a) of housing (12) for coupling syringe (1) with infusion device (10), said ring nut (14) being interrupted to allow inserting syringe (1) on base (12a) of housing (12), and defining with said base (12a) a pair of diametrically opposite slots (16) where tabs (7) of syringe (1) can be inserted by rotating body (3) of syringe (1) about its axis;
**characterized in that** the syringe (1) further has, in correspondence of base (3a), at least one locator (9) arranged to abut against the side wall (14a) of said ring nut (14) when syringe (1) is properly positioned, with tabs (7) properly located in slots (16), whereby said at least one locator (9) prevents any further rotation of body (3) of syringe (1)..

2. The combination as claimed in claim 1, wherein said syringe (1) has asymmetrical tables (7)

3. The combination as claimed in claim 1 or 2, wherein said at least one locator comprises a stop tooth (9) in correspondence of said base (3a) of said body (3).

4. The combination as claimed in claim 3, wherein said syringe (1) comprises a pair of stop teeth (9), each associated with one tab (7).

5. The combination as claimed in claim 4, wherein said stop teeth (9) are located at diametrically opposite positions relative to said body (3).

6. The combination as claimed in claim 4 or 5, wherein each stop tooth (9) is located adjacent to the corresponding tab (7).

7. The combination as claimed in any of claims 4 to 6, wherein each said tab (7) has a draft portion (7b), distal from said stop tooth (9), and a stopping portion (7c), proximal to said stop tooth (9), said draft portion (7b) and said stopping portion (7c) having different profiles.

8. The combination as claimed in claim 7, wherein said draft portion (7b) has a greater radius of curvature (r_{b}) and therefore a more smoothed and rounded profile, and wherein said stopping portion (7c) has a smaller radius of curvature (r_{c}) and therefore a sharper profile.

9. The combination as claimed in any of claims 3 to 7, wherein said at least one stop tooth (9) is integrally formed with said body (3).

10. The combination as claimed in any of claims 3 to 7, wherein said at least one stop tooth (9) is applied, for instance glued or soldered, to said body (3).

## Patentansprüche

1. Kombination einer Injektionsvorrichtung (10) und einer Spritze (1),
- wobei die Spritze (1) aufweist:
- einen im Wesentlichen zylindrischen Körper (3), der mit einem flüssigen Medikament gefüllt sein kann, wobei der Körper (3) an einem ersten Ende oder einer Basis (3a) ein Paar Laschen (7) zum Ankoppeln der Spritze durch Verdrehen an die Infusionsvorrichtung (10) und an einem entgegengesetzten Ende (3b) einen Anschluss zum Anbringen einer Nadel oder einer Kanüle zum Verabreichen des flüssigen Medikaments aufweist;
- wobei die Infusionsvorrichtung (10) aufweist:
- ein Gehäuse (12), in dem ein Elektromotor und entsprechende mechanische Mittel zum axialen Verschieben eines Schiebers, der den Stößel der Spritze (1), die in der Infusionsvorrichtung (10) montiert ist und ein Medikament enthält, verschiebt, um nach und nach das in der Spritze enthaltene Medikament zu verabreichen, sowie eine elektronische Steuervorrichtung, die dazu vorgesehen ist, die Rate und die Dauer der Verabreichung des Medikaments und andere Parameter der Infusionsvorrichtung einzustellen, angeordnet ist;
- eine Ringmutter (14), die in Verbindung mit einer Basis (12a) des Gehäuses (12) zum Koppeln der Spritze (1) mit der Infusionsvorrichtung (10) vorgesehen ist, wobei die Ringmutter (14) unterbrochen ist, um das Einsetzen der Spritze (1) in die Basis (12a) des Gehäuses (12) zu ermöglichen, und mit der Basis (12a) ein Paar diametral entgegengesetzter Schlitze (16) definiert, in die Laschen (7) der Spritze (1) eingeführt werden können, indem der Körper (3) der Spritze (1) um seine Achse gedreht wird;
**dadurch gekennzeichnet, dass** die Spritze (1) zudem im Zusammenwirken mit der Basis (3a) wenigstens einen Positionsanzeiger (9) aufweist, der dafür vorgesehen ist, an die Seitenwand (14a) der Ringmutter (14) anzuschlagen, wenn die Spritze (1) mit richtig in den Schlitzen (16) sitzenden Laschen (7) korrekt positioniert ist, wodurch der wenigstens eine Positionsanzeiger (9) eine weitere Drehung des Körpers (3) der Spritze (1) verhindert.

2. Kombination nach Anspruch 1, wobei die Spritze (1) asymmetrische Laschen (7) hat.

3. Kombination nach Anspruch 1 oder 2, wobei der wenigstens eine Positionsanzeiger einen Stoppzahn (9) aufweist, der mit der Basis (3a) des Körpers (3) zusammenwirkt.

4. Kombination nach Anspruch 3, wobei die Spritze (1) ein Paar Stoppzähne (9) aufweist, die jeweils einer Lasche (7) zugeordnet sind.

5. Kombination nach Anspruch 4, wobei die Stoppzähne (9) an diametral gegenüberliegenden Position relativ zu dem Körper (3) angeordnet sind.

6. Kombination nach Anspruch 4 oder 5, wobei jeder Stoppzahn (9) angrenzend an die entsprechende Lasche (9) angeordnet ist.

7. Kombination nach einem der Ansprüche 4 bis 6, wobei jede Lasche (7) distal von dem Stoppzahn (9) ein Zugabschnitt (7b) und proximal zu dem Stoppzahn (9) ein Stoppabschnitt (7c) aufweist, wobei der Zugabschnitt (7b) und der Stoppabschnitt (7c) verschiedene Profile haben.

8. Kombination nach Anspruch 7, wobei der Zugabschnitt (7b) einen größeren Krümmungsradius (r_{b}) und deshalb ein glatteres und runderes Profil hat und wobei der Stoppabschnitt (7c) einen kleineren Krümmungsradius (r_{c}) und deshalb ein schärferes Profil hat.

9. Kombination nach einem der Ansprüche 3 bis 7 wobei der wenigstens ein Stoppzahn (9) integral mit dem Körper (3) ausgebildet ist.

10. Kombination nach einem der Ansprüche 3 bis 7 wobei wenigstens ein Stoppzahn (9) an dem Körper (3) angebracht ist, beispielsweise angeklebt oder angelötet.

## Revendications

1. Combinaison d'un dispositif d'injection de médicament (10) et d'une seringue (1),
- dans laquelle la seringue (1) comprend :
- un corps sensiblement cylindrique (3) qui peut être rempli d'un médicament liquide, ledit corps (3) ayant au niveau d'une première extrémité ou base (3a), une paire de languettes (7) pour coupler la seringue au dispositif d'injection (1) par rotation et, à l'extrémité opposée (3b), une borne (5) pour monter une aiguille ou une canule pour administrer ledit médicament liquide ;
- dans laquelle le dispositif d'injection (10) comprend :
- un logement (12) qui accueille un moteur électrique et des moyens mécaniques correspondants pour déplacer axialement un poussoir qui pousse le piston de la seringue (1) montée dans ledit dispositif d'injection (10) et contenant un médicament de manière à délivrer graduellement ledit médicament contenu dans la seringue, ainsi qu'un dispositif de commande électronique agencé pour ajuster la cadence et la durée de distribution de médicament et d'autres paramètres du dispositif d'injection ;
- un écrou à oeillet (14) ménagé en correspondance avec une base (12a) du logement (12) pour coupler la seringue (1) avec le dispositif d'injection (10), ledit écrou à oeillet (14) étant interrompu pour permettre l'insertion de la seringue (1) sur la base (12a) du logement (12), et définissant avec ladite base (12a) une paire de fentes diamétralement opposées (16) où des languettes (7) de la seringue (1) peuvent être insérées en faisant tourner le corps (3) de la seringue (1) autour de son axe ;
**caractérisée en ce que** la seringue (1) comporte, en correspondance de la base (3a), au moins un localisateur (9) agencé pour venir en butée contre la paroi latérale (14a) dudit écrou à oeillet (14) lorsque la seringue (1) est positionnée correctement, avec des languettes (7) localisées correctement dans des fentes (16), moyennant quoi ledit au moins un localisateur (9) empêche toute rotation supplémentaire dudit corps (3) de la seringue (1).

2. Combinaison selon la revendication 1, dans laquelle ladite seringue (1) comporte des languettes asymétriques (7) .

3. Combinaison selon la revendication 1 ou 2, dans laquelle ledit au moins un localisateur comprend une dent d'arrêt (9) en correspondance de ladite base (3a) dudit corps (3).

4. Combinaison selon la revendication 3, dans laquelle ladite seringue (1) comprend une paire de dents d'arrêt (9), chacune associée à une languette (7).

5. Combinaison selon la revendication 4, dans laquelle lesdites dents d'arrêt (9) sont localisées à des positions diamétralement opposées relativement audit corps (3).

6. Combinaison selon la revendication 4 ou 5, dans laquelle chaque dent d'arrêt (9) est localisée adjacente à la planguette correspondante (7).

7. Combinaison selon l'une quelconque des revendications 4 à 6, dans laquelle chaque dite languette (7) comporte une portion de dérive (7b), à distance de ladite dent d'arrêt (9), et une portion d'arrêt (7c), à proximité de ladite dent d'arrêt (9), ladite portion de dérive (7b) et ladite portion d'arrêt (7c) ayant des profils différents.

8. Combinaison selon la revendication 7, dans laquelle ladite portion de dérive (7b) a un rayon de courbure (r_{b}) plus grand et donc un profil plus lissé et arrondi, et dans laquelle ladite portion d'arrêt (7c) a un rayon de courbure (r_{c}) plus petit et donc un profil plus acéré.

9. Combinaison selon l'une quelconque des revendications 3 à 7, dans laquelle ladite au moins une dent d'arrêt (9) est formée solidairement avec ledit corps (3).

10. Combinaison selon l'une quelconque des revendications 3 à 7, dans laquelle ladite au moins une dent d'arrêt (9) est appliquée, par exemple collée ou soudée, audit corps (3) .
